# EUROPÄISCHE PATENTANMELDUNG

(11) **EP 0 792 631 A1**
(43) Veröffentlichungstag der Anmeldung: **03.09.1997**
(21) Anmeldenummer: 97102204.1
(22) Anmeldetag: 12.02.1997
(51) Int. Cl.: A61J 15/00, A61M 39/10

(54) **Sondenanordnung für die enterale Ernährung und/oder zum Absaugen von Sekretansammlungen im Magen**

(30) Priorität: 28.02.1996 DE 29603624 U
(71) Anmelder: Fresenius AG, 61350 Bad Homburg v.d.H (DE)
(72) Erfinder: Speitling, Anette, 61476 Kronberg (DE)
(74) Vertreter: Fuchs, Luderschmidt & Partner

(57) **Zusammenfassung**

Eine Sondenanordnung für die enterale Ernährung und/oder zum Absaugen von Sekretansammlungen im Magen des Patienten besteht aus einem flexiblen Schlauch (1), der mit einem hülsenförmigen Anschlußstück (2) versehen ist, einem Adapterstück (11), das auf das hülsenförmige Anschlußstück (2) des Schlauchs aufgesetzt werden kann und einem in den Schlauch einführbaren Spiralmandarin (20), der mit einem hülsenförmigen Endstück (22) versehen ist, das auf das Adapterstück (11) aufsetzbar ist. Das Adapterstück (11) weist einen passend in das Anschlußstück (2) einsteckbaren rohrförmigen Anschlußstutzen (12) auf, an den sich ein Kegelschaft (13) anschließt, der von einem rohrförmigen mit einem Innengewinde (17) versehenen Teil (14) konzentrisch umgeben ist. Das rohrförmige Teil (14) ist mit seinem einen Ende an einem flanschförmigen Teil (15) angeschlossen, das seinerseits mit dem rohrförmigen Anschlußstutzen (12) des Adapterstücks (11) verbunden ist. Der Kegelschaft (13) bildet zusammen mit dem rohrförmigen Teil (14) das eine Verbindungsteil einer verriegelbaren Kegelverbindung (Luer-Lock-Verbindung) und erlaubt den Anschluß der bekannten Überleitgeräte an den Schlauch (1) der Sonde. Für den Fall, daß die enterale Ernährung mittels der bekannten Blasenspritzen durchgeführt werden soll oder Sekretansammlungen im Magen des Patienten mittels der bekannten Blasenspritzen abgesaugt werden sollen, wird das Adapterstück (11) einfach von dem hülsenförmigen Anschlußstück (2) abgezogen, so daß sich der Ansatz der Blasenspritzen auf das hülsenförmige Anschlußstück (2) aufsetzen läßt.

## Beschreibung

Die Erfindung betrifft eine Sondenanordnung für die enterale Ernährung und/oder zum Absaugen von Sekretansammlungen im Magen des Patienten.

Die bekannten Sonden zur enteralen Ernährung können an ein Überleitgerät angeschlossen werden, über das die flüssige Nahrung in die Sonde geleitet wird. Zur Konnektierung des Überleitgerätes ist der Schlauch der bekannten Ernährungssonden mit einem Verbindungsteil versehen, das zusammen mit dem Verbindungsteil des angeschlossenen Überleitgerätes eine verriegelbare Kegelverbindung (Luer-Lock-Verbindung) bildet. Als nachteilig erweist sich, daß die bekannten Sonden mit positivem Luer-Lock-Ansatz dann nicht verwendet werden können, wenn die enterale Ernährung mittels der bekannten Blasenspritzen durchgeführt werden soll oder Sekretansammlungen aus dem Magen des Patienten mittels der bekannten Blasenspritzen abgesaugt werden sollen. In diesem Fall muß auf Sonden mit einem hülsenförmigen Anschlußstück zurückgegriffen werden, das sich mit dem Ansatz der Blasenspritzen verbinden läßt.

Der Erfindung liegt die Aufgabe zugrunde, den Einsatzbereich einer Sonde für die enterale Ernährung und/oder zum Absaugen von Sekretansammlungen derart zu vergrößern, daß sich die Sonde an verschiedene Geräte auf einfache Weise anschließen läßt.

Die Lösung der Aufgabe erfolgt erfindungsgemäß mit den Merkmalen des Patentanspruchs 1.

Die erfindungsgemäße Anordnung besteht aus einem flexiblen Schlauch, der mit einem hülsenförmigen Anschlußstück versehen ist, einem Adapterstück, das auf das hülsenförmige Anschlußstück des Schlauchs aufgesetzt werden kann und einem in den Schlauch einführbaren Spiralmandarin, der mit einem hülsenförmigen Endstück versehen ist, das auf das Adapterstück aufsetzbar ist. Das Adapterstück weist einen passend in das hülsenförmige Anschlußstück einsteckbaren rohrförmigen Anschlußstutzen auf, an den sich ein Kegelschaft anschlielßt, der von einem rohrförmigen, mit einem Innengewinde versehenen Teil konzentrisch umgeben wird. Das rohrförmige Teil ist mit seinem einen Ende an einem flanschförmigen Teil angeschlossen, das seinerseits mit dem rohrförmigen Anschlußstutzen des Adapterstücks verbunden ist. Der Kegelschaft bildet zusammen mit dem im wesentlichen rohrförmigen Teil das eine Verbindungsteil einer verriegelbaren Kegelverbindung (Luer-Lock-Verbindung). Zum Anschluß der bekannten Überleitgeräte an den Schlauch der Sonde wird das Adapterstück auf das Anschlußstück des Schlauchs aufgesetzt. Für den Fall, daß die enterale Ernährung mittels der bekannten Blasenspritzen durchgeführt werden soll oder Sekretansammlungen im Magen des Patienten mittels der bekannten Blasenspritzen abgesaugt werden sollen, wird das Adapterstück einfach von dem hülsenförmigen Anschlußstück abgezogen, so daß sich der Ansatz der Blasenspritze passend in die im wesentlichen zylindrische Ausnehmung des hülsenförmigen Anschlußstücks einschieben läßt. Das Adapterstück der erfindungsgemäßen Ernährungssonde erlaubt es also, verschiedene Geräte mit einer einzigen Sonde zu verbinden. Daher ist eine Vorratshaltung von verschiedenen Sonden mit verschiedenen Anschlußstücken nicht erforderlich.

Der Schlauch mit dem Anschlußstück der Sonde kann aus Silikon, Polyurethan oder PVC bestehen.

In einer bevorzugten Ausführungsform weist die Ernährungssonde ein zweites Adapterstück auf, das über einen flexiblen Verbindungssteg unverlierbar mit dem hülsenförmigen Anschlußstück verbunden ist. Das zweite Adapterstück kann anstelle des ersten Adapterstücks auf das hülsenförmige Anschlußstück des Schlauchs aufgesetzt werden, so daß sich eine Luer-Spritze mit der Ernährungssonde verbinden läßt. Das zweite Adapterstück weist hierzu einen passend in das hülsenförmige Anschlußstück einsteckbares hülsenförmiges Teil auf, an das sich ein flanschförmiges Teil mit einem dem konischen Anschlußstück der Luer-Spritze komplementären Innenkonus anschließt.

Um den Innenkonus des zweiten Adapterstücks verschließen zu können, ist ein Verschlußteil mit entsprechender Konizität vorgesehen, der über einen weiteren flexiblen Verbindungssteg unverlierbar mit dem zweiten Adapterstück verbunden ist.

Das zweite Adapterstück und/oder der Verschlußteil sind vorzugsweise mit einer Grifflasche versehen, um diese Teile leicht abziehen zu können.

Wenn vor dem Gebrauch der Ernährungssonde nur das Endstück des Spiralmandarins von dem Adapterstück abgeschraubt und der Spiralmandarin aus dem Schlauch herausgezogen wird, steht eine Sonde mit positivem Luer-Lock-Ansatz zur Verfügung, auf den der negative Luer-Lock-Ansatz der bekannten Überleitgeräte aufgesetzt werden kann. Wird der Spiralmandarin zusammen mit dem ersten Adapterstück aus dem Anschlußstück herausgezogen, steht eine Sonde mit einem hülsenförmigen Anschlußstück zur Verfügung, das sich mit dem Ansatz der bekannten Blasenspritzen verbinden läßt. Auf das hülsenförmige Anschlußstück kann bei Bedarf das zweite Adapterstück aufgesetzt werden, das mit dem kegelstumpfförmigen Verschlußteil verschlossen werden kann.

Nachfolgend wird unter Bezugnahme auf die Zeichnungen ein Ausführungsbeispiel der Erfindung näher erläutert.

Es zeigen:
- Fig. 1: den mit dem hülsenförmigen Anschlußstück versehenen Schlauch der Sondenanordnung für die enterale Ernährung und/oder zum Absaugen von Sekretansammlungen im Magen des Patienten,
- Fig. 2: einen Längsschnitt durch das hülsenförmige Anschlußstück,
- Fig. 3: einen Längsschnitt durch das auf das hülsenförmige Anschlußstück aufsetzbare erste Adapterstück,
- Fig. 4: das erste Adapterstück in der Seitenansicht,
- Fig. 5: den Spiralmandarin in geschnittener Darstellung, der mit einem auf das erste Adapterstück aufsetzbaren Endstück versehen ist,
- Fig. 6: eine Ansicht des hülsenförmigen Anschlußstücks aus der Richtung des Pfeils VI von Fig. 2 und
- Fig. 7: einen Schnitt entlang der Linie VII-VII von Fig. 6.

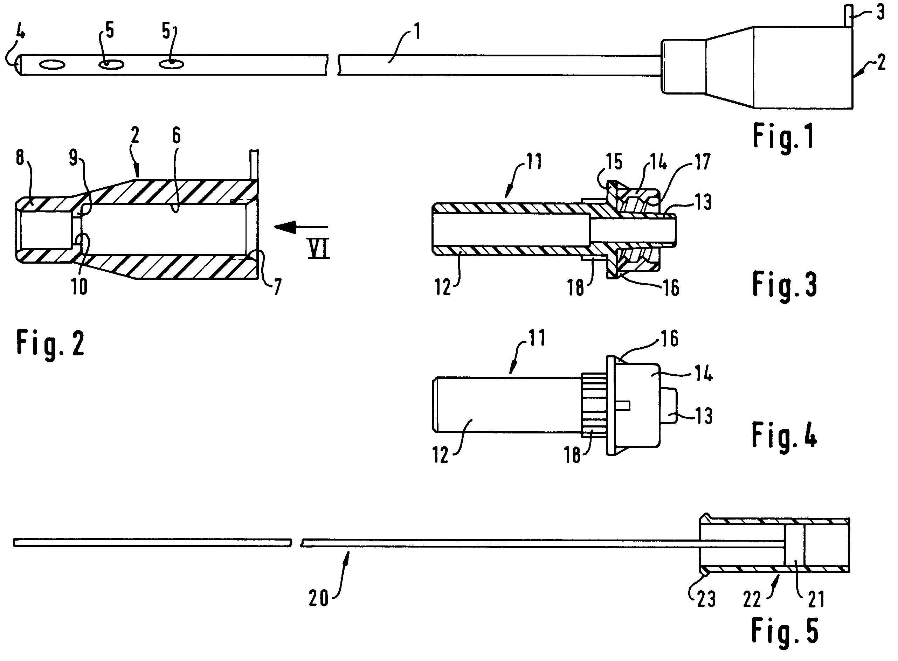

Die erfindungsgemäße Sondenanordnung für die enterale Ernährung und/oder zum Absaugen von Sekretansammlungen im Magen des Patienten besteht aus einem mit einem hülsenförmigen Anschlußstück versehenen flexiblen Schlauch (Fig. 1), einem auf das hülsenförmige Anschlußstück aufsetzbaren ersten Adapterstück (Fig. 4) und einem in den flexiblen Schlauch einführbaren Spiralmandarin, der mit einem auf das erste Adapterstück aufsetzbaren Endstück versehen ist (Fig. 5).

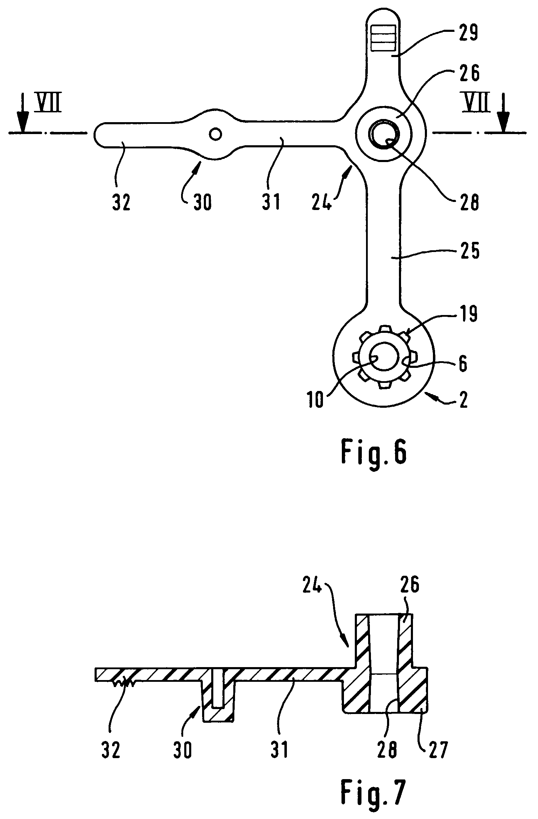

Fig. 1 zeigt den flexiblen Schlauch 1 der Sonde zusammen mit dem hülsenförmigen Anschlußstück 2, das auf das distale Ende des Schlauchs aufgesetzt und mit diesem verschweißt ist. Das Anschlußstück 2 ist mit einem Adapterstück versehen, das über einen in Fig. 1 nur teilweise dargestellten flexiblen Verbindungssteg 3 mit dem Anschlußstück 2 verbunden ist. Das in Fig. 1 nicht dargestellte Adapterstück wird nachfolgend noch unter Bezugnahme auf die Fign. 6 und 7 beschrieben. Der Schlauch 1 und das Anschlußstück 2 bestehen aus Silikon. Das proximale Ende des Schlauchs 1 ist mit einem Verschlußstopfen 4 verschlossen, wobei der proximale Endbereich des Schlauchs 1 mit mehreren Ausnebmungen 5 versehen ist, aus denen die dem Patienten über die Sonde zugeführte Nahrung austreten kann.

Fig. 2 zeigt einen Längsschnitt durch das hülsenförmige Anschlußstück 2 des Schlauchs 1. Das Anschlußstück 2 ist nach Art eines Trichters ausgebildet und weist eine im wesentlichen zylindrische Ausnehmung 6 auf, in die entweder der Ansatz der bekannten Blasenspritzen oder ein Adapterstück eingesetzt werden kann, das unter Bezugnahme auf die Fign. 3 und 4 noch beschrieben wird. Der innere Randbereich 7 des hülsenförmigen Anschlußstücks 2 ist konusförmig erweitert. An seinem rückwärtigen Ende ist das hülsenförmige Anschlußstück 2 mit einem rohrförmigen Ansatz 8 versehen, der das distale Schlauchende passend aufnimmt. Der mittlere Steg 9 zwischen den beiden zylindrischen Ausnehmungen ist von einer zentralen Durchgangsbohrung 10 durchbrochen.

Fig. 3 zeigt das Adapterstück 11 zum Anschluß des Überleitungsschlauches der bekannten Überleitgeräte. Das Adapterstück weist einen rohrförmigen Anschlußstutzen 12 auf, der passend in die zylindrische Ausnehmung 6 des hülsenförmigen Anschlußstücks 2 eingesteckt werden kann, so daß die Teile dicht miteinander verbunden sind. Um eine dichte Verbindung von Anschlußstück 2 und Adapterstück 11 sicherzustellen, kann es vorteilhaft sein, wenn sich der rohrförmige Anschlußstutzen 12 zu seinem freien Ende hin geringfügig erweitert. An den rohrförmigen Anschlußstutzen 12 schließt sich ein Kegelschaft 13 an, der von einem rohrförmigen Teil 14 konzentrisch umgeben ist, das mit seinem einen Ende an einem flanschförmigen Teil 15 des Adapterstücks 11 angeschlossen ist. Das rohrförmige Teil 14 stützt sich mit mehreren umfangsmäßig verteilt angeordneten Rippen 16 an dem flanschförmigen Teil 15 des Adapterstücks 11 ab. Die Länge des rohrförmigen Anschlußstutzens 12 ist so bemessen, daß das freie Ende des Anschlußstutzens an dem mittleren Steg 9 des hülsenförmigen Anschlußstücks 2 und der flanschförmige Teil 15 des Adapterstücks 11 an dem vorderen Rand des Anschlußstücks 2 anliegt, sofern das Adapterstück 11 vollständig in das Anschlußstück eingeschoben ist. Der Außendurchmesser des flanschförmigen Teils 15 des Adapterstücks 11 entspricht dem Außendurchmesser des hülsenförmigen Anschlußstücks 2.

Das rohrförmige Teil 14 des Adapterstücks 11 ist mit einem Innengewinde 17 mit zwei Gängen versehen und bildet zusammen mit dem Kegelschaft 13 das eine Verbindungsteil einer verriegelbaren Kegelverbindung (Luer-Lock-Verbindung). Der Überleitungsschlauch der bekannten Überleitgeräte ist mit einem entsprechenden als Kegelhülse ausgebildeten Ansatz versehen, wobei der Innenkonus der Kegelhülse (negativer Luer-Lock-Ansatz) mit dem Außenkonus des Kegelschaftes 13 des Adapterstücks 11 komplementär ist (positiver Luer-Lock-Ansatz).

Im Bereich des flanschförmigen Teils 15 ist der hülsenförmige Anschlußstutzen 12 des Adapterstücks 11 leicht verbreitert und mit einer rippenartigen Profilierung 18 versehen. Der konusförmig erweiterte innere Randbereich 7 des hülsenförmigen Anschlußstücks 2 ist mit einer entsprechenden Profilierung 19 versehen (Fig. 6). Der rohrförmige Anschlußstutzen 12 des Adapterstücks 11 kann zunächst mit seinem freien Ende lose in die konusförmige Erweiterung des hülsenförmigen Anschlußstücks 2 eingesetzt und dann unter leichter Aufweitung des Anschlußstücks vollständig eingeschoben werden.

Fig. 5 zeigt den Spiralmandarin 20, der durch das Adapterstück 11 und das hülsenförmige Anschlußstück 2 in den flexiblen Schlauch 1 eingeführt wird. Der Spiralmandarin 20 ist mit einem mittleren Steg 21 eines hülsenförmigen Endstücks 22 verbunden. Das Endstück 22 wird auf das Adapterstück 11 aufgesetzt und mit dem positiven Luer-Lock-Ansatz des Adapterstücks verschraubt. Dabei greifen die Nasen 23 des hülsenförmigen Endstücks 22 in die Gewindegänge 17 des Adapterstücks 11.

Um auf das hülsenförmige Anschlußstück 2 auch die Ansätze der bekannten Luer-Spritzen aufsetzen zu können, ist das Anschlußstück 2 mit einem zweiten Adapterstück 24 versehen. Das zweite Adapterstück 24 ist über einen flexiblen Verbindungssteg 25 am äußeren Rand des hülsenförmigen Anschlußstücks 2 befestigt (Fig. 6). Es weist einen passend in das hülsenförmige Anschlußstück 2 einsteckbares hülsenförmiges Teil 26 auf, an das sich ein flanschförmiger Teil 27 mit einem dem Ansatz der Luer-Spritze entsprechenden Innenkonus 28 anschließt (Fig. 7). Das hülsenförmige Teil 26 des zweiten Adapterstücks 24 kann ferner passend auf den Kegelschaft 13 des ersten Adapterstücks 11 aufgesteckt werden, so daß diese Teile dicht miteinander verbunden sind. Daher ist es möglich, eine Luer-Spritze mit der Sonde sowohl bei aufgesetztem ersten Adapterstück 11 als auch bei abgenommenen Adapterstück 11 zu verbinden. Das zweite Adapterstück 24 ist mit einer Grifflasche 29 versehen. Ferner ist ein kegelstumpfförmiges Verschlußteil 30 mit dem zweiten Adapterstück 24 verbunden. Dieser ist an dem zweiten Adapterstück 24 über einen weiteren Verbindungssteg 31 angeschlossen, der sich im rechten Winkel zu dem Verbindungssteg 25 des zweiten Adapterstücks 24 erstreckt und in einer Grifflasche 32 endet. Der kegelstumpfförmige Verschlußteil 30 weist eine dem Innenkonus 28 des zweiten Adapterstücks 24 entsprechende Konizität auf, so daß das zweite Adapterstück 24 mit dem Verschlußteil 30 dicht verschlossen werden kann.

## Patentansprüche

1. Sondenanordnung für die enterale Ernährung und/oder zum Absaugen von Sekretansammlungen im Magen bestehend aus einem flexiblen Schlauch (1), der im Bereich seines proximalen Endes mit mindestens einer Öffnung (5) und an seinem distalen Ende mit einem hülsenförmigen Anschlußstück (2) versehen ist, einem Adapterstück (11), das einen passend in das hülsenförmige Anschlußstück (2) einsteckbaren im wesentlichen rohrförmigen Anschlußstutzen (12) aufweist, an den sich ein Kegelschaft (13) anschließt, wobei der Kegelschaft (13) von einem mit einem Innengewinde (17) versehenen rohrförmigen Teil (14) konzentrisch umgeben ist, das mit seinem einen Ende an einem flanschförmigen Teil (15) angeschlossen ist, welches mit dem rohrförmigen Anschlußstutzen (12) verbunden ist und einem in den Schlauch (1) einführbaren Spiralmandarin (20), der mit einem hülsenförmigen Endstück (22) versehen ist, das auf das erste Adapterstück (11) aufsetzbar ist.

2. Sondenanordnung nach Anspruch 1, **dadurch gekennzeichnet**, daß ein zweites Adapterstück (24) über einen flexiblen Verbindungssteg (25) mit dem hülsenförmigen Anschlußstück (2) verbunden ist, wobei das zweite Adapterstück (24) ein passend in das hülsenförmige Anschlußstück (2) einsteckbares hülsenförmiges Teil (26) umfaßt, an das sich ein flanschförmiges Teil (27) mit einem Innenkonus (28) anschließt.

3. Sondenanordnung nach Anspruch 2, **dadurch gekennzeichnet**, daß mit dem zweiten Adapterstück (24) über einen weiteren flexiblen Verbindungssteg (31) ein kegelförmiger Verschlußteil verbunden ist, der in den Innerkonus (28) des flanschförmigen Teils (27) des zweiten Adapterstücks (24) passend einsteckbar ist.

4. Sondenanordnung nach Anspruch 3, **dadurch gekennzeichnet**, daß das zweite Adapterstück (24) und/oder der Verschlußteil (30) mit einer Grifflasche (29, 32) versehen ist.

5. Sondenanordnung nach einem der Ansprüche 1 bis 4, **dadurch gekennzeichnet**, daß der Außendurchmesser des flanschförmigen Teils (15) des ersten Adapterstücks (11) dem Außendurchmesser des hülsenförmigen Anschlußstücks (2) entspricht.
